# EUROPEAN PATENT SPECIFICATION

(11) **EP 2 555 798 B1**
(45) Date of publication and mention of the grant of the patent: **22.06.2016**
(21) Application number: 11764957.4
(22) Date of filing: 11.04.2011
(51) Int. Cl.: A61K 39/42, A61P 31/18, C07K 16/04, C07K 16/10

(54) **METHODS AND COMPOSITIONS FOR INHIBITING HIV TRANSMISSION**
VERFAHREN UND ZUSAMMENSETZUNGEN ZUR HEMMUNG VON HIV-ÜBERTRAGUNGEN
PROCÉDÉS ET COMPOSITIONS POUR L'INHIBITION DE LA TRANSMISSION DU VIH

(30) Priority: 09.04.2010 US 322399 P
(43) Date of publication of application: 13.02.2013
(73) Proprietor: Reef Pharmaceuticals Pty Ltd, South Yarra, VIC 3141 (AU)
(72) Inventor: RAWLIN, Grant, Thomas, Kilmore East Victoria 3764 (AU); PURCELL, Damian, Francis, John, North Balwyn Victoria 3104 (AU); CENTER, Robert, John, Rosanna Victoria 3084 (AU); KRAMSKI, Marit, Middle Park Victoria 3206 (AU); ROBINS-BROWNE, Roy, Michael, Templestowe Victoria 3106 (AU); LICHTI, Gottfried, Essendon Victoria 3040 (AU)
(74) Representative: Walker, Ross Thomson
(86) International application number: PCT/AU2011/000407
(87) International publication number: WO 2011/123900

(56) References cited:
- WO-A1-2009/058989
- WO-A1-2010/096042
- WO-A2-2008/063331
- US-A- 6 039 957
- KRAMSKI M ET AL: "P13-03. Neutralizing antibody to HIV-1 Env from hyperimmune bovine colostrum as a mucosal microbicide strategy for preventing virus transmission", RETROVIROLOGY, BIOMED CENTRAL LTD., LONDON, GB, vol. 6, no. Suppl 3, 22 October 2009 (2009-10-22), page P185, XP021063889, ISSN: 1742-4690, DOI: 10.1186/1742-4690-6-S3-P185
- J. P. NKOLOLA ET AL: "Breadth of Neutralizing Antibodies Elicited by Stable, Homogeneous Clade A and Clade C HIV-1 gp140 Envelope Trimers in Guinea Pigs", JOURNAL OF VIROLOGY, vol. 84, no. 7, 1 April 2010 (2010-04-01), pages 3270-3279, XP055083632, ISSN: 0022-538X, DOI: 10.1128/JVI.02252-09
- M. KRAMSKI ET AL: "Hyperimmune Bovine Colostrum as a Low-Cost, Large-Scale Source of Antibodies with Broad Neutralizing Activity for HIV-1 Envelope with Potential Use in Microbicides", ANTIMICROBIAL AGENTS AND CHEMOTHERAPY, vol. 56, no. 8, 1 August 2012 (2012-08-01) , pages 4310-4319, XP055083604, ISSN: 0066-4804, DOI: 10.1128/AAC.00453-12
- MARIT KRAMSKI ET AL: "Anti-HIV-1 antibody-dependent cellular cytotoxicity mediated by hyperimmune bovine colostrum IgG", EUROPEAN JOURNAL OF IMMUNOLOGY, vol. 42, no. 10, 28 October 2012 (2012-10-28), pages 2771-2781, XP055083789, ISSN: 0014-2980, DOI: 10.1002/eji.201242469
- CENTER R J ET AL: "Promoting trimerization of soluble human immunodeficiency virus type 1 (HIV-1) Env through the use of HIV-1/simian immunodeficiency virus chimeras", JOURNAL OF VIROLOGY, THE AMERICAN SOCIETY FOR MICROBIOLOGY, US, vol. 78, no. 5, 1 March 2004 (2004-03-01), pages 2265-2276, XP002317305, ISSN: 0022-538X, DOI: 10.1128/JVI.78.5.2265-2276.2004
- ZHANG P. F. ET AL.: 'Extensively cross-reactive anti-HIV-1 neutralizing antibodies induced by gp140 immunization' PNAS vol. 104, no. 24, 2007, pages 10193 - 10198
- DU S.X. ET AL.: 'Effect oftrimerization motifs on quaternary structure, antigenicity, and immunogenicity of a non-cleavable HIV-1 gp140 envelope glycoprotein' VIROLOGY vol. 395, no. 1, 2009, pages 33 - 44
- HAYNES B.F. ET AL.: 'Aiming to induce broadly reactive neutralizing antibody responses with HIV-1 vaccine candidates' EXPERT. REV. VACCINES vol. 5, no. 3, 2006, pages 347 - 363
- Mikyung Kim ET AL: "Comparison of HIV Type 1 ADA gp120 Monomers versus gp140 Trimers as Immunogens for the Induction of Neutralizing Antibodies", AIDS Research and Human Retroviruses, vol. 21, no. 1, 1 January 2005 (2005-01-01), pages 58-67, XP055177893, ISSN: 0889-2229, DOI: 10.1089/aid.2005.21.58
- CENTER R J ET AL: "Induction of HIV-1 subtype B and AE-specific neutralizing antibodies in mice and macaques with DNA prime and recombinant gp140 protein boost regimens", VACCINE, ELSEVIER LTD, GB, vol. 27, no. 47, 5 November 2009 (2009-11-05), pages 6605-6612, XP026722062, ISSN: 0264-410X, DOI: 10.1016/J.VACCINE.2009.08.016 [retrieved on 2009-08-25]
- GERALD V. QUINNAN ET AL: 'Immunization of Rabbits with Highly Purified, Soluble, Trimeric Human Immunodeficiency Virus Type 1 Envelope Glycoprotein Induces a Vigorous B Cell Response and Broadly Cross-Reactive Neutralization' PLOS ONE vol. 9, no. 5, E98060, 01 January 2014, pages 1 - 14, XP055177897 DOI: 10.1371/journal.pone.0098060 ISSN: 1932-6203

## Description

### Field

The present invention provides methods useful in the field of medicine, and particularly in the treatment of viral infections. More particularly, the disclosure relates to the use of methods and compositions for the inhibition of human immunodeficiency virus (HIV) transmission. Background

The retrovirus designated human immunodeficiency virus (HIV) is the etiological agent of the complex disease that includes progressive destruction of the immune system (acquired immune deficiency syndrome; AIDS) and degeneration of the central and peripheral nervous system. Since it emerged as a public health threat in the early 1980's, efforts to control or eradicate the disease have focused principally on options for treating the disease after an individual has already become infected.

The use of condoms provides a substantial degree of protection against transmission of HIV infections during sexual intercourse. However, the use of condoms is not 100% effective against the transmission of HIV. Moreover, couples often do not use condoms. A topical composition that could be inserted into the vagina or rectum by a foam, gel, sponge or other form, or which could be topically applied to the male genitalia, would in many cases be preferred over condoms. Moreover, the prophylactic effectiveness of condoms could be improved by including a suitable microbicide in the lubricant coated on the exterior of the condom. However, to date little progress has been made to develop an effective topical composition against the transmission of HIV. Most work to develop topical HIV prophylactic compositions has focused on the use of surfactants and buffers, such as the over-the-counter product nonoxynol-9. Surfactants and detergents disrupt microbial and sperm membranes by lysis and emulsification. Surfactant-containing creams and gels have the advantage of being very broad in their killing ability, and thus can kill the HIV virus and viruses associated with other sexually transmitted diseases. The use of surfactants and buffers is, however, substantially limited by the damage they can cause to cell membranes. In the vagina, nonoxynol-9 has been shown to thin vaginal walls. In the rectum, nonoxynol-9 can cause rectum walls to slough off.

Other virusidal compositions being investigated for use as HIV virusides include carageenan and other large sulfated polysaccharides that stick to viral envelopes and possibly shield cell membranes. Non-nucleoside inhibitors of the human immunodeficiency virus reverse transcriptase have also been shown to have some effect against IIIV.

Scientists have recently reported several biological discoveries that improve our understanding of how H IV enters an organism following sexual contact, which could lead to prophylactic substances that interfere with HIV's interaction with its target cells. These discoveries revolve generally around T lymphocytes, monocytes/macrophages and dendritic cells, suggesting that CD4 cell receptors arc engaged in the process of virus transmission. For example, it is thought that HIV tightly binds the surface of dendritic cells, and when the dendritic cells present microbial antigens to CD4+ T helper cells to stimulate an immune response, the dendritic cell inadvertently transfers the HIV to the CD4+ T cells, thereby advancing the progression of the infection.

Some have postulated, based upon these discoveries, that prophylactics can be designed that block the interaction between the virus and the human host. However, methods that rely on the specific interaction of HIV and human cells are limited, because the infection pathway has not been fully defined and may be diverse. (Miller, C. J. et al., "Genital Mucosal Transmission of Simian Immunodeficiency Virus: Animal Model for Heterosexual Transmission of Human Immunodeficiency Virus", J. Virol., 63, 4277-4284 (1989); Phillips, D. M. and Bourinbaiar, A. S., "Mechanism of HIV Spread from Lymphocytes to Epithelia", Virology, 186, 261 -273 (1992); Phillips, D. M., Tan, X., Pearce- Pratt, R. and Zacharopoulos, V. R., "An Assay for II IV Infection of Cultured Human Cervix-derived Cells", J. Virol. Methods, 52, 1-13 (1995); Ho, J. L. et al., "Neutrophils from Human Immunodeficiency Virus (HIV)-SeronegatiVe Donors Induce HIV Replication from HIV-infected Patients Mononuclear Cells and Cell lines": An In Vitro Model of HIV Transmission Facilitated by Chlamydia Trachomatis., "J. Exp. Med., 181 , 1493-1505 (1995); and Braathen, L. R. & Mork, C. in "IIIV infection of Skin Langerhans Cells", In: Skin Langerhans (dendritic) cells in virus infections and AIDS (ed. Becker, Y.) 131-139 (Kluwer Academic Publishers, Boston, (1991)).

Efforts by researchers to develop an HIV vaccine have also not yet been successful. For example, vaccination with inactivated SIV docs not protect African Green monkeys against infection with the homologous virus notwithstanding a strong immune response to SIV.

Kramski M. et al: "P13-03. Netralizing antibody to HIV-1 Env from hyperimmune colostrum as a mucosal microbicide strategy for preventing virus transmission", Retrovirology, Biomed Central Ltd., vol 6, no Suppl 3, 22 October 2009, page 185, discloses the immunization of pregnant cows with HIV-1 Env gp140 oligomers to yield high titre HIV Env polyclonal antibodies concentrated into colostrum.

As will be apparent from the foregoing review of the prior art, there remain significant problems to be overcome in the prevention and treatment of HIV and HIV transmission. It is an aspect of the present disclosure to overcome or ameliorate a problem of the prior art by providing compositions and methods for the inhibition of HIV transmission. The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention as it existed before the priority date of each claim of this application.

### Summary of the Invention

According to the invention, there is provided a non-therapeutic method for producing polyclonal antibodies or fragments thereof capable of binding to a human immunodeficiency virus (HIV) viral envelope (Env) protein or a fragment thereof, the method comprising:
immunizing a female cow with oligomeric Clade R gp140; and
obtaining the antibodies or fragments thereof from hyperimmune colostrum of the cow,
the method characterised by the steps of:
   administering a primary vaccination of the oligomeric Clade B gp140 to the cow during a pregnancy; and
   administering two booster vaccinations of the oligomeric Clade B gp140 to the cow during the pregnancy;
   wherein the first booster vaccination is administered 7 weeks after the primary vaccination and the second booster vaccination is administered 12 weeks after the primary vaccination.

Preferably the Clade B oligomeric gp140 comprises gp140 trimers, dimers and monomers.

Advantageously the animal is immunized with the HIV viral envelope (Env) protein or fragment thereof and an adjuvant.

Conveniently the adjuvant is a water-in-oil emulsion.

### Brief Description of the Drawings

Figure 1 illustrates a gp140 vaccination schedule.
Figure 2 illustrates a gp140 vaccination schedule. Figure 3 demonstrates IgG from serum and colostrum binds to gap140 Env of clade A, B and C.
Figure 4 demonstrates purified colostrum IgG from non-pregnant cows retains binding to gp140 Env and demonstrates heterologous binding activity.

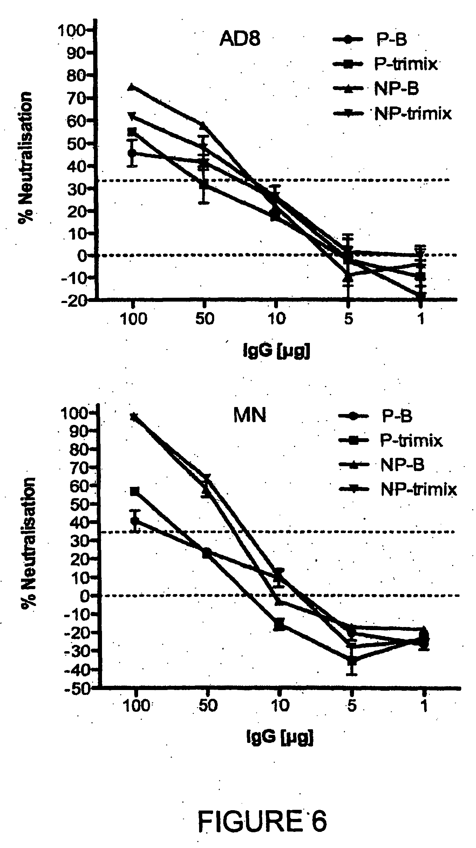

Figure 5 demonstrates bovine IgG blocks binding of monoclonal Ab b12 to CD4 binding site of gp140 Figure 6 demonstrates colostrum from pregnant cows vaccinated with clade A B/C gp140 and non-pregnant cows vaccinated with clade B gp140 have broad heterologous neutralizing activity. Figure 7 demonstrates purified colostrum IgG has neutralizing activity.

### Detailed Description of the Invention

The present invention is predicated in part on the finding that highly specific colostrum antibodies binding to the HIV Env protein can be generated by vaccination of pregnant animals. Accordingly, in a first aspect the present disclosure provides a composition for inhibiting transmission of HIV comprising polyclonal antibodies or fragments thereof capable of binding to a human immunodeficiency virus (HIV) viral envelope (Env) protein or a fragment thereof. The Env protein or fragment thereof may be any HIV Env protein, however preferably the Env protein is gp140. Without wishing to be limited by theory, it is proposed that the polyclonal antibodies act to bind HIV virions, thereby inhibiting movement ofHIV through cells that form the barrier layer on mucosal surfaces. In one aspect the composition is capable of inhibiting or preventing HIV infection of a cell. In another aspect the composition is capable of inhibiting or preventing HIV movement through epithelial cells, such as those that form the barrier layer on mucosal surfaces. This approach to formulating compositions and method for inhibiting transmission of HIV is distinguished from approaches of the prior art, and is indeed contrary to the general teaching of the prior art prior to the present disclosure.

The term "inhibiting transmission" as used herein, generally refers to complete inhibition and also partial inhibition of HIV transmission. Complete inhibition indicates that the HIV virus is completely unable to successfully infect and/or replicate and/or further infect other cells. This can be determined in a number of ways, at the cellular and/or whole organism level, by the skilled practitioner. One such determination is by an inability to obtain infectious HIV from a host cell. Another such determination is by an inability to determine that IIIV has entered the host cell. At the whole organism level, standard methods for assaying for HIV infection can be used (e.g., assaying for antibodies to HIV in the individual). Partial inhibition refers to a measurable, statistically significant reduction in the ability of HIV to infect and/or replicate and/or further infect other cells, as compared to an appropriate control which has not been subjected to the therapeutics described herein. One example would be a requirement for higher levels of exposure or longer period of exposure to HIV for successful infection.

The term "capable of binding" as used herein, generally refers to an antibody that binds to a gp140 of a clade of HIV, such as an antibody described herein. Binding to a gp140 of a clade of HIV may be demonstrated as described in the Examples below. In usefulaspects , the antibodies or fragments thereof bind to a gp140 of a strain or clade of HI V the antibodies are raised against, and also bind to a gp 140 of a strain or clade of HIV the antibodies are not raised against. In other useful aspects, the antibodies or fragments thereof bind to a gp140 of the clade of HIV the antibodies are raised against, and also bind to a gp140 of a heterologous clade of H IV.

The term "clade(s)", as used herein, generally encompasses subtypes or recombinant forms of HIV.

Previous work has indicated there is a need to provide improved compositions that provide protection against HIV and/or inhibition of transmission of HIV. In particular, there is a need to provide compositions that protect against HIV without compromising the integrity of the innate protective surface layer of the vagina or rectum. Work towards this end has focused on active immunity (e.g. vaccines), however when antibodies against HIV are used in compositions against HIV, these antibodies are made using HIV antigen which would difficult to achieve regulatory approval for and use due to a risk of infection and difficulty of manufacture in volume. In contrast to the teachings of the prior art, the present invention is predicated in part on the provision of passive hetero-immunity, wherein antibodies made in a particular organism are used to protect another organism, generally a different species. The Env ectodomain is known as gp140, which contains both gp120 and truncated gp41 (lacking transmembrane domains and cytoplasmic tails). In one aspect, the Env protein or fragment thereof is a gp140 oligomer. The oligomer may comprise gp140 trimers, dimers and monomers. The Oligomers may be purified from transduced cell (e.g. HeLa and 293) supernatant, for example by lentil lectin affinity chromatography and gel filtration. The Env protein or fragment thereof may be a HIV clade A, clade B or clade C strain viral envelope (Env) protein or fragment thereof. In a related application (PCT/AU2009/001218), Applicants have demonstrated strains of HIV-1 have differences in their Envs.

In one aspect the composition comprises polyclonal antibodies or fragments thereof capable of binding to a Clade A, Clade B or Clade C gp140.

In another aspect the composition comprises polyclonal antibodies or fragments thereof capable of binding to a Clade A and a Clade B gp140. In another aspectthe composition comprises polyclonal antibodies or fragments thereof capable of binding to a Clade B and a Clade C gp140.

In another aspect the composition comprises polyclonal antibodies or fragments thereof capable of binding to a Clade A and a Clade C gp140.

In another aspect the composition comprises polyclonal antibodies or fragments thereof capable of binding to a Clade A, a Clade B and a Clade C gp140. In one aspect the composition comprises polyclonal antibodies or fragments thereof raised against Clade B gp140 that are capable of binding to a heterologous Clade A, Clade B or Clade C gp140. In one aspect, the composition comprises polyclonal antibodies or fragments thereof that compete with monoclonal antibody Ab b12 binding to gp140. In another aspect, the composition comprises polyclonal antibodies or fragments thereof raised against Clade B gp140 that are capable of binding to a heterologous Clade A. Clade B or Clade C gp140, wherein the antibodies or fragments thereof compete with monoclonal antibody Ab b12 binding to gp140.

Surprisingly, Applicants have demonstrated colostrum, and IgG purified from colostrum, from cows vaccinated with HIV Env gp140 oligomers of one clade can bind HIV Env gp140 of another clade, despite diversity in Env sequence and glycosylation across HTV strains. Furthermore, applicants have demonstrated colostrum, and IgG purified from colostrum, from cows vaccinated with HIV Env gp140 oligomers of one clade can bind gp140 and neutralize HIV of another clade, despite diversity in Env sequence and glycosylation across HIV strains. Dairy cows were vaccinated in the second trimester of pregnancy with high quality soluble oligomeric HIV-1 Env (gp140) to produce colostrum containing high levels of HIV-1 Env-specific polyclonal neutralizing antibodies for use as an HIV transmission inhibiting composition. The present inventors have shown that anti-HIV Env IgG synergizes with intrinsic antiviral components in bovine colostrum to aggressively neutralize HIV-1.

Accordingly, the present disclosure provides an advantage of the production of kilogram quantities of bovine IgG. Without wishing to be bound by theory, the compositions of the present disclosure are proposed to have potent ability to neutralise HIV and thereby render it non-infectious for susceptible cells in vitro.

In oneaspect, the polyclonal antibodies or fragments thereof are neutralizing antibodies. The term "neutralisation" as used herein, generally refers to antibodies or fragments thereof that are able to bind the molecule of the disclosure and hamper its biological activity. The term encompasses antibodies or fragments thereof that block a virus, e.g. H IV, from infecting a cell by, for example, blocking gp140 binding to CD4 on a cell.

The antibody, or fragment thereof, or functional equivalent thereof may be produced by immunization of an animal with recombinant p140. The animal may be immunized with a clade B gp140 and the antibodies produced are capable of binding to a heterologous Clade A, Clade B or Clade C gp140.

The animal may be immunized with Clade B gp140 and antibodies produced that are capable of binding to a heterologous Clade A, Clade B or Clade C gp140.

The animal may be immunized with Clade B gp140 and antibodies produced compete with monoclonal antibody Ab b12 binding to gp140.

The animal may be immunized with Clade B gp140 and antibodies produced that are capable of binding to a heterologous Clade A, Clade B or Clade C gp140, wherein the antibodies produced compete with monoclonal antibody Ab b12 binding to gp140.

In one aspect, the polyclonal antibodies or fragments thereof produced are neutralizing antibodies. In one aspect, the polyclonal antibodies or fragments thereof produced block binding of gp140 to CD4 on a cell.

In one aspect, the recombinant gp140 may not be derived from virion culture.

An Env polypeptide that is suitable to generate an immune response is an Env polypeptide having at least 90%, 95%, 96%, 97%, 98%, 99% or 100% amino acid identity to gp140. gp140 contains a fragment of a gp120 from a given HIV strain and a fragment of gp41 from the same H IV strain, wherein the soluble gp41 fragment lacks the transmembrane domain. gp140 polypeptides capable of forming oligomeric structures may be expressed from a construct. Accordingly, there is provided a nucleotide sequence that encodes for a gp140 polypeptide, and an expression construct comprising a nucleotide sequence that codes for a gp140 polypeptide. The expression construct may be one for transient use or be more suitable for stable transfection and maintenance within a target cell as either an episomally replicating construct or an integrated form. In one aspectthere is provided a gp140 polypeptide, and an expression construct that expresses a gp140 polypeptide. In anotheraspect, there is provided a glycosylated gp140 polypeptide, which has been manufactured or expressed in an expression system that adds the native cellular glycosylation.

Recombinant gp140 may be produced using pseudoviruses carrying Env from different clades/strains using the expression vectors included in Table 1. gp140 may be purified by a number of different means. For example, gp140- containing tissue culture supernatants may be passed over lentil lectin affinity columns, which mediate the capturing of glycoproteins, including gp140, through the affinity of lentil lectin for carbohydrate. After washing, gp140 is eluted competitively from the column by the addition of 0.5M Methyl -D- mannopyranoside (Sigma). Yields obtained with this system for other gp140 strains have varied between 0.4 and 1 .0 milligram per 100 millilitres of tissue culture supernatant. The eluate may then be concentrated and further purified by gel filtration over superdex 200. The gp140 is purified from the culture supernatants in an oligomeric form.

Soluble Env gp140 oligomers have been prepared from clade A, B, and C HIV-1 strains from HeLa and/or 293T cells and purified by lentil lectin affinity and gel filtration chromatography. Four cows (two pregnant in second semester and two initially non-pregnant) were vaccinated with 100 g of purified HIV-1 Env gp140 oligomer formulated with Montanide adjuvant. Two groups of two cows (one pregnant and one nonpregnant) were vaccinated with either clade B (AD8) only or with equal amounts (33.33pg) of clade A, B and C Env gp140 (UG8, AD8 and MW) (referred to as 'trimix'). All four cows received at least three vaccinations whereas the last vaccination was given four weeks before giving birth. All four cows seroconverted within nine weeks. Reciprocal endpoint serum IgG titers were up to 1 x10^{2 5} for pregnant cows and up to 1 x10⁵ for non-pregnant cows determined by a new established anti-bovine IgG HIV-1 Env gp140 specific ELISA. The expected low serum IgG titer in pregnant cows was explained by the pumping of serum IgG antibodies into the colostrum approximately four weeks before giving birth.

HIV-immune bovine colostrum was collected and pasteurised postpartum from all cows with pregnancy vaccination resulting in relatively low responses with reciprocal IgG titers of < 10² (clade B vaccinated) and 1 x 10^{3 5} (trimix- vaccinated). Reciprocal colostrum IgG titer for cows vaccinated before pregnancy was 10⁵ (clade B vaccinated) and 10^{4 5} (trimix vaccinated). Western blot analysis confirmed that colostrum IgG of all four cows was specific against HIV-1 Env gp140. Unfractionated colostrum was tested for neutralising activity in a HIV-1 Env-pseudotyped reporter virus assay.

In brief, Env-pseudotyped reporter virus assay detects the presence of virus-neutralising antibodies to HIV-1 envelope protein. EGFP reporter pseudovirus particles expressing HIV-1 Env derived from strains/clades of choice are used to infect target cells in an Env dependent manner. In one assay, the reporter pseudovirus particles are incubated for 1 hour before the addition of the target cells (Cf2th-CD4/CCR5/CXCR4; CF2 cells) at 2x10⁴/well in a 96-well plate. After a 2-hour spinoculation at 1200 x g at room temperature, residual pseudovirus and antibody was removed and fresh media added to the cells. Two days later the target cells are analysed for EGFP expression by FACS. In the presence of colostrum or colostrum IgG raised against soluble HIV-1 Env gp140 oligomers, the degree of reduction in the level of infection was determined by measuring the reduction in the percent EGFP positive cells. The neutralisation percentage represents a ratio between infection levels observed in mice sera before vaccination (pre-bleed) and mice sera 2 weeks post protein boost 3 vaccination. Clade A/E, clade B and clade C pseudotype viruses including the NIH reference panel for clade B and C viruses were tested (total n=27) and compared with non-immune bovine colostrum that already has intrinsic infection-blocking activity due to lactoferrin and other bioactive peptides. Unfractionated colostrum from the trimix cow vaccinated during pregnancy showed high neutralisation of up to 50% for all B clade pseudoviruses (n=15) as well as for the majority of C clade (n=1 1) and clade A/E (n=1) pseudoviruses at a dilution of 1 :16. The first clade B vaccinated cow was a low responder but both cows vaccinated before pregnancy and having their calves recently responded well. Up to this time, broad neutralisation was observed for the clade B vaccinated cow that showed 50-80% neutralisation for B clade (n=12) and clade C pseudoviruses (n=9) (1:16 dilution) (Tablel). IgG Abs from the first pair of cows was purified from the colostrum and neutralising activity was retained for purified IgG with up to 50% neutralisation for the trimix- IgG compared to non immune IgG at 500 g/ml. Results of the neutralisation profile of two HIV Env gp140 hyperimmune bovine colostrum samples against pseudoviruses of different clades are demonstrated in Table 1 (see, Example 2). This result is surprising since antibodies raised against gp140 are not expected to bind and neutralize viruses of different clades strongly, since there are significant epitope differences between the gp140 of the different clades.

These results strongly support this method of raising high levels of neutralising antibodies, and neutralizing antibodies that can bind heterologous HIV strains. Accordingly, in oneaspect, the polyclonal antibodies or fragments thereof are capable of binding to an Env protein from a heterologous clade of HIV or a heterologous strain of HIV. In one aspect the composition comprises polyclonal antibodies or fragments thereof capable of binding to a Clade A, Clade B or Clade C gp140.

In another aspectthe composition comprises polyclonal antibodies or fragments thereof capable of binding to a Clade A and a Clade B gp140.

In another aspectthe composition comprises polyclonal antibodies or fragments thereof capable of binding to a Clade B and a Clade C gp140.

In another aspectthe composition comprises polyclonal antibodies or fragments thereof capable of binding to a Clade A and a Clade C gp140.

In another aspectthe composition comprises polyclonal antibodies or fragments thereof capable of binding to a Clade A, a Clade B and a Clade C gp140.

In another aspectthe composition comprises polyclonal antibodies or fragments thereof raised against Clade B gp140 that are capable of binding to a heterologous Clade A, Clade B or Clade C gp140. In another aspectthe composition comprises polyclonal antibodies or fragments thereof that compete with monoclonal antibody Ab b12 binding to gp140. In anotheraspect, the polyclonal antibodies or fragments thereof are neutralizing antibodies.

In another aspectthe composition comprises polyclonal antibodies or fragments thereof raised against Clade B gp140 that are capable of binding to a heterologous Clade A, Clade B or Clade C gp140, wherein the polyclonal antibodies or fragments thereof compete with monoclonal antibody Ab b12 binding to gp140. The antibody, or fragment thereof, or functional equivalent thereof may be present in or obtained from an avian egg, or present in or obtained from hyperimmune colostrum or hyperimmune milk of an animal. The animal may be a cow. Methods for generating hyperimmune sera, milk, colostra and the like are known in the art.

The method for generating the hymperimmune material may comprise the step of purifying the Env protein from other potentially immunogenic molecules. Four example, Env proteins can isolated by methods such as high and low speed centrifugation, optionally with the use of gradients formed using sucrose, percoll, cesium and the like. Chromotagraphic methods such as size exclusion chromatography, affinity chromatography, high performance liquid chromatography, reverse phase chromatography, and the like are also useful. Electrophoretic methods (such as capillary electrophoresis), filtration methods (such as tangential flow ultrafiltration), partitioning methods (such as protein precipitation) are further examples of useful methods. Chronically infected cell lines may be developed by infection of cells, e.g. 6D5 cells (a subelone of the HUT78 cell line) with HIV. Radioimmunoprecipitation analysis is used to examine that the cell line secretes Env into the medium. The Env protein may then be purified from the serum-free conditioned medium by affinity chromatography using mouse MAbs to the Env protein. For the production of hyperimmune material, the Env protein (whether or not purified) is administered to an animal, typically by way of injection (for example, via the IM, subcutanteous, intraperitoneal, or intravenous route). The Env protein may be combined with an adjuvant to increase the immune response generated by the animal.

Accordingly, the animal may be immunized with a HIV viral envelope (Env) protein or a fragment thereof and an adjuvant. In one aspect, the adjuvant is a water in oil emulsion.

The skilled person is familiar with many potentially useful adjuvants, such as Freund's complete adjuvant, alum, and squalene. Adjuvants which may be used in compositions of the disclosure include, but are not limited to oil emulsion compositions suitable for use as adjuvants in the disclosure include oil-in- water emulsions and water-in-oil emulsions, complete Freund's adjuvant (CFA) and incomplete Freund's adjuvant (IFA) may also be used. Preferably, Montanide brand adjuvants may be used (e.g. MONTANIDE ISA 50V, MONTANIDE ISA 206, and MONTANIDE IMS 1312). These adjuvants are oily adjuvant compositions of mannide oleate and mineral oil, or water based nanoparticles combined with a soluble immunostimulant.

Adjuvants suitable for include bacterial or microbial derivatives such as derivatives of enterobacterial lipopolysaccharide (LPS), Lipid A derivatives, immunostiinulatory oligonucleotides and ADP-ribosylating toxins and detoxified derivatives thereof.

The animal may be dosed with Env at intervals over a period of days, weeks or months. At the conclusion of the immunization regime, the hyperimmune material (such as blood, milk or colostrums) is harvested. Antibodies in the hyperimmune material may be harvested by any suitable method, including any by method described supra.

In one aspect the composition comprises antibodies from colostrum or a colostrum extract, further characterised in that the colostrum is enriched in anti-Env antibodies when compared with colostrum obtained without vaccination.

In one aspect of the method the polyclonal antibodies are obtained from a hyperimmune material. The hyperimmune material is enriched when compared with corresponding material in which the animal has not been challenged with the antigen in question.

The animal used to produce the hyperimmune material may be any suitable animal, including a human. However, since human milk may contain potentially transmissible human pathogens, one form of the method provides that the antibody is not human-derived. In any event, animals that produce large quantities of milk are preferred. In this regard, ungulates (and cows in particular), are animals useful for the generation of hyperimmune material.

In one aspect of the method, the "hyperimmune material" is hyperimmune dairy derived material such as milk particularly colostral milk (colostrum) and the like which is enriched in antibodies or fragments thereof and which is derived from an animal source. The hyperimmune dairy material is preferably hyperimmune colostrum.

In another aspect the hyperimmune material is derived from bird eggs. A subtype of immunoglobulin known as IgY can be easily extracted from the yolk. Typically, the yolk is first defatted and the IgY isolated by methods identical or similar to those used for skim milk.

The term "colostrum" as used herein includes colostral milk; processed colostral milk such as colostral milk processed to partly or completely remove one or more of fat, cellular debris, lactose and casein; and colostral milk or processed colostral milk which has been dried by for example, freeze drying, spray drying or other methods of drying known in the art. Colostral milk is generally taken from a mammal such as a cow within five days after parturition. Preferably the mammalian colostrum is bovine colostrum retained from the first 4 days post parturition, more preferably bovine colostrum retained from the first 2 days post parturition, even more preferably bovine colostrum retained from the first day post parturition, and most preferably bovine colostrum retained from the first milking post parturition. Preferably the colostrum collected from the cow comprises at least 4% total protein (weight %), more preferably 5%, more preferably at least 8%, more preferably at least 10%.

Preferably the ratio of IgG to total protein of the colostrum collected from the cow is at least 10%, more preferably 20%.

The hyperimmune dairy material preferably contains at least 3 g per kilogram of product which is TgG directed against Env, or an equivalent molar concentration of the anti-Env antibody. For example the hyperimmune material may contain at least 5g, at least 10g or at least 15g anti-Env antibody per kg of hyperimmune material on the basis of the dry weight of components. The upper end of the range of antibody concentration will depend on factors such as the dose, the disease state and the health of the patient. The hyperimmune material may, for example contain no more than 80g such as no more than 60g, no more than 50g or no more than 40g anti-Env antibody per kg of hyperimmune material on the basis of the dry weight of components.

In one aspect of the method the polyclonal antibodies are administered to the subject as a composition. The composition may in one aspect comprise a carrier admixed with the ligand prior to administration, for example, by mixing a composition of hyperimmune colostrum from immunized cows or one or more processed components thereof with conventional foods and/or pharmaceutically acceptable excipients. The ratio of enriched product relative to conventional dairy material from unvaccinated animals may, for example, be at least 4, such as at least 10 in a comparative ELISA assay.

In another aspect part or all of the antibodies specific for Env are extracted from the colostrum and used to prepare a composition for administration. In one aspect the hyperimmune material binds Env derived from a clade A, clade B or clade C HIV-1 strain. Preferably the hyperimmune material binds at least two of the above, more preferably at least 3 of the clades. The degree of enrichment in material selected from antibodies capable of binding to Env may be at least 4 times, for example at least 10 times the level found in corresponding unvaccinated animals with respect each of the Env molecules as determined by standard ELISA. In one aspect, low molecular weight moieties have been substantially removed from the colostrum or the colostrum extract. By substantially removed is meant that at least 75% and preferably 90% of the low molecular weight moieties are removed. In a preferred example of this aspect at least 75% (such as at least 90% or substantially complete removal) of, moieties of molecular weight less than 30kDa have been removed from the colostrum or the colostrum extract. Preferably molecular weight moieties less than 60kDa have been substantially removed from the colostrum or colostrum extract.

In one aspect, the hyperimmune material comprises immunogenic material selected from antibody and antibody fragments which bind Env. Preferably the antibody or antibody fragment is a polyclonal antibody or a polyclonal antibody fragment of bovine origin.

The composition may further contain growth factor molecules that are normally found in milk or colostrum. These factors may produce a synergism with the anti-Env antibodies contained in the composition. Exemplary growth factors include TGF-beta-1 , TGF-beta-2, IGF-1 , IGF-2, EGF, FGF and PDGF.

The composition may be formulated for topical administration, and in certain aspect the composition is formulated for vaginal or rectal administration. The composition may be formulated as a gel, or formulated as a topical cream, ointment, lotion or foam formulation. The topical formulations of the present disclosure can be used to prevent HIV infection in a human, or to inhibit transmission of the HIV virus from an infected human to another human. The topical formulations of the present disclosure can inhibit the growth or replication of a virus, such as a retrovirus, in particular a human immunodeficiency virus, specifically HIV-1 and HIV-2. The topical formulations arc useful in the prophylactic treatment of humans who are at risk for viral infection. The topical formulations also can be used to treat objects or materials, such as contraceptive devices (for example condoms or intrauterine devices), medical equipment, supplies, or fluids, including biological fluids, such as blood, blood products, and tissues, to prevent or inhibit viral infection of a human. Such topical formulations also are useful to prevent transmission, such as sexual transmission of viral infections, e.g., HIV, which is the primary way in which HIV is transmitted globally. The methods of prevention or inhibition or retardation of transmission of viral infection, e.g., HIV infection, in accordance with the present disclosure, comprise vaginal, rectal, penile or other topical treatment with an antiviral effective amount of a topical preparation of the present disclosure, alone or in combination with another antiviral compound as described herein.

Preferred compositions can take several forms. Thus, in one aspect the composition is in the form of a cream, lotion, gel, or foam that is applied to the affected skin or epithelial cavity, and preferably spread over the entire skin or epithelial surface which is at risk of contact with bodily fluids. Such formulations, which are suitable for vaginal or rectal administration, may be present as aqueous or oily suspensions, solutions or emulsions (liquid formulations) containing in addition to the active ingredient, such carriers as are known in the art to be appropriate. For "stand-alone" lubricants (i.e., lubricants that are not pre-packaged with condoms), gels and similar aqueous formulations are generally preferred, for various reasons (both scientific and economic) known to those skilled in the art. These formulations are useful to protect not only against sexual transmission of HIV, but also to prevent infection of a baby during passage through the birth canal. Thus the vaginal administration can take place prior to sexual intercourse, during sexual intercourse, and immediately prior to childbirth.

One method of applying an anti-viral lubricant to the genitals, for the purposes disclosed herein, involves removing a small quantity (such as a teaspoon, or several milliliters) of a gel, cream, ointment, emulsion, or similar formulation from a plastic or metallic tube, jar, or similar container, or from a sealed plastic, metallic or other packet containing a single dose of such composition, and spreading the composition across the surface of the penis immediately before intercourse. Alternate methods of emplacement include: (1) spreading the composition upon accessible surfaces inside the vagina or rectum shortly before intercourse; and (2) emplacing a condom, diaphragm, or similar device, which has already been coated or otherwise contacted with an anti-viral lubricant, upon the penis or inside the vagina. In a preferred aspect, any of these methods of spreading an anti-viral lubricant across the surfaces of the genitals causes the lubricant to coat and remain in contact with the genital and epithelial surfaces throughout intercourse.

In another aspect, the present disclosure involves topical administration of the topical formulation to the anus. The composition administered to the anus is suitably a foam or gel, etc., such as those described above with regard to vaginal application. In the case of anal application, it may be preferred to use an applicator which distributes the composition substantially evenly throughout the anus. For example, a suitable applicator is a tube 2.5 to 25 cm, preferably 5 to 10 cm, in length having holes distributed regularly along its length.

When the composition is a water-soluble vaginal cream or gel, suitably 0.1 to 4 grams, preferably about 0.5 to 2 grams, are applied. When the composition is a vaginal spray-foam, suitably 0.1 to 2 grams, preferably about 0.5 to 1 grams, of the spray-foam are applied. When the composition is an anal cream or gel, suitably 0.1 to 4 grams, preferably about 0.5 to 2 grams of the cream or gel is applied. When the composition is an anal spray-foam, suitably 0.1 to 2 grams, preferably about 0.5 to 1 grams of the spray-foam are applied. As a vaginal formulation, the active ingredient may be used in conjunction with a spermicide and may be employed with a condom, diaphragm, sponge or other contraceptive device. Examples of suitable spermicides include nonylphenoxypolyoxyethylene glycol (nonoxynol 9), benzethonium chloride, and chlorindanol. Suitably, the pH of the composition is 4.5 to 8.5. Vaginal compositions preferably have a pH of 4.5 to 6, most preferably about 5.

Vaginal formulations also include suppositories (for example, gel- covered creams), tablets and films. The suppositories can be administered by insertion with an applicator using methods well known in the art.

Typical buccal formulations are creams, ointments, gels, tablets or films that comprise ingredients that are safe when administered via the mouth cavity. Buccal formulations can also comprise a taste-masking or flavoring agent.

The present compositions may also be in the form of a time-release composition. In this aspect, the composition is incorporated in a composition which will release the active compound at a rate which will result in the vaginal or anal concentration described above. Time- release compositions are disclosed in Controlled Release of Pesticides and Pharmaceuticals, D. H. Lew, Ed., Plenum Press, New York, 1981 ; and U.S. Pat. Nos. 5,185,155; 5,248,700; 4,01 1,312; 3,887,699; 5,143,731 ; 3,640,741 ; 4,895,724; 4,795,642; Bodmeier et al, Journal of Pharmaceutical Sciences, vol. 78 (1989); Amies, Journal of Pathology and Bacteriology, vol. 77 (1959); and Pfister et al, Journal of Controlled Release, vol. 3, pp. 229-233 (1986).

The present compositions may also be in the form which releases the composition in response to some event such as vaginal or anal intercourse. For example, the composition may contain the anti_ent antibodies in vesicles or liposomes which are disrupted by the mechanical action of intercourse. Compositions comprising liposomes are described in U.S. Pat. No. 5,231 ,1 12 and Deamer and Uster, "Liposome Preparation: Methods and Mechanisms", in Liposomes, pp. 27-51 (1983); Sessa et al, J. Biol. Chem., vol. 245, pp. 3295-3300 (1970); Journal of Pharmaceutics and Pharmacology, vol. 34, pp. 473-474 (1982); and Topics in Pharmaceutical Sciences, D. D. Breimer and P. Speiser, Eds., Elsevier, New York, pp. 345-358 (1985)
It should also be realized that the present compositions may be associated with a contraceptive device or article, such as a vaginal ring device, an intrauterine device (IUD), vaginal diaphragm, vaginal sponge, pessary, condom, etc. In the case of an IUD or diaphragm, time-release and/or mechanical-release compositions may be preferred, while in the case of condoms, mechanical-release compositions are preferred.

A suitable vaginal ring drug delivery system for slow release of the anti-Env antibodies is disclosed in US Patent 5,989,581. As described in U.S. Pat. No. 5,989,581 , the vaginal ring delivers two actives for contraception. The drug delivery system disclosed comprises at least one compartment comprising a drug dissolved in a thermoplastic polymer core and a thermoplastic skin covering the core. Preferred thermoplastic polymers for both the core and the skin are ethylene- vinylacetate copolymers. As would be understood by one skilled in the art, according to the present disclosure, the disclosed delivery system contains at anti-Env antibodies useful to prevent, inhibit or slow infection or transmission of HIV. In certain aspects, said vaginal ring device may also contain one or more additional drugs, for instance a contraceptive agent such as a steroidal progestogenic compound and/or a steroidal estrogenic compound. In yet other aspects, the vaginal ring system containing a anti-Env antibodies may also contain or be used in combination with a topical estriol, such as Ovestin™, to enhance prevention of infection or transmission of HIV through the vaginal epithelium.

In another aspect, the present disclosure provides novel articles which are useful for the prevention or retardation of HIV infection. In particular, the present articles are those which release anti-Env antibodies when placed on an appropriate body part or in an appropriate body cavity. Thus, the present article may be a vaginal ring device as described above or an ILID. Suitable ILIDs are disclosed in U.S. Pat. Nos. 3,888.975 and 4,283,325.

The present article may be an intravaginal sponge which comprises and releases, in a time-controlled fashion, the anti-Env antibodies. Intravaginal sponges are disclosed in U.S. Pat. Nos. 3,916,898 and 4,360,013,. The present article may also be a vaginal dispenser which releases the anti-Env antibodies. Vaginal dispensers are disclosed in U.S. Pat. No. 4,961 ,931.

In one aspect the compositions are used in conjunction with condoms, to enhance the risk-reducing effectiveness of condoms and provide maximum protection for users. The composition can either be coated onto condoms during manufacture, and enclosed within conventional watertight plastic or foil packages that contain one condom per package, or it can be manually applied by a user to either the inside or the outside of a condom, immediately before use.

As used herein, "condom" refers to a barrier device which is used to provide a watertight physical barrier between male and female genitalia during sexual intercourse, and which is removed after intercourse. This term includes conventional condoms that cover the penis; it also includes so-called "female condoms" which are inserted into the vaginal cavity prior to intercourse. The term "condom" does not include diaphragms, cervical caps or other barrier devices that cover only a portion of the epithelial membranes inside the vaginal cavity. Preferably, condoms should be made of latex or a synthetic plastic material such as polyurethane, since these provide a high degree of protection against viruses.

In another aspect the compositions are used in conjunction with other possible surfaces for transmission, such as gloves, to provide maximum protection for users. The composition can either be coated onto gloves during manufacture, and enclosed within conventional watertight plastic or foil packages that contain one pair of gloved per package, or it can be manually applied by a user to either the inside or the outside of a glove, immediately before use.

In another aspect the composition is in the form of an intra-vaginal pill, an intra-rectal pill, or a suppository. The suppository or pill should be inserted into the vaginal or rectal cavity in a manner that permits the suppository or pill, as it dissolves or erodes, to coat the vaginal or rectal walls with a prophylactic layer of the anti-HIV agent.

In still another aspect the composition is topically applied by release from an intravaginal device. Devices such as vaginal rings, vaginal sponges, diaphrams, cervical caps, female condoms, and the like can be readily adapted to release the composition into the vaginal cavity after insertion.

In certain aspects, the composition may further comprise a pharmaceutically acceptable excipient, a lubricant, or an antiviral agent.

Compositions used in the methods of this disclosure may also comprise other active agents, such as another agent to prevent HIV infection, and agents that protect individuals from conception and other sexually transmitted diseases. Thus, in another aspect the compositions used in this disclosure further comprise a second anti-HIV agent, a virucide effective against viral infections other than HIV, and/or a spermicide.

In one particular aspect, the composition contains nonoxynol, a widely- used spermicidal surfactant. The resulting composition could be regarded as a "bifunctional" composition, since it would have two active agents that provide two different desired functions, in a relatively inert carrier liquid; the nonoxynol would provide a spermicidal contraceptive agent, and the polyclonal antibodies or fragments thereof would provide anti-viral properties. The nonoxynol is likely to cause some level of irritation, in at least some users; this is a regrettable but is a well-known side effect of spermicidal surfactants such as nonoxynol and octoxynol, which attack and destroy the lipid bilayer membranes that surround sperm cells and other mammalian cells. The compositions used in this disclosure may also contain a lubricant that facilitates application of the composition to the desired areas of skin and epithelial tissue, and reduces friction during sexual intercourse. In the case of a pill or suppository, the lubricant can be applied to the exterior of the dosage form to facilitate insertion.

In still another aspect the disclosure provides a device for inhibiting the sexual transmission of HIV comprising (a) a barrier structure for insertion into the vaginal cavity, and (b) a composition comprising a polyclonal antibody according to the present disclosure- As mentioned above, preferred devices which act as harrier structures, and which can be adapted to apply anti-HIV agent, include the vaginal sponge, diaphram, cervical cap, or condom (male or female).

In the cream or ointment aspects of the present disclosure, the topical formulation comprises one or more lubricants. The gels and foams of the present disclosure optionally can include one or more lubricants.

Non-limiting examples of useful lubricants include cetyl esters wax, hydrogenated vegetable oil, magnesium stearate, methyl stearate, mineral oil, polyoxyethylene-polyoxypropylene copolymer, polyethylene glycol, polyvinyl alcohol, sodium lauryl sulfate, white wax, or mixtures of two or more of the above.

The amount of lubricant in the topical formulation can range from about 0 to about 95 weight percent. Typical cream and ointment formulations comprise 0.1 to 95 weight percent of lubricant.

The topical formulations can comprise one or more adjutants, wherein the adjuvant is an antimicrobial agent, antioxidant, humectant or emulsifier, or mixture of two or more thereof. The gels and foams of the present disclosure can include one or more antimicrobial agents and optionally can include one or more of antioxidants, humectants and emulsifiers. Non-limiting examples of useful antimicrobial agents are benzyl alcohol, propylene glycol, propyl paraben, methyl paraben, or mixtures of two or more thereof.

The amount of antimicrobial agents in the topical formulation can range from about 0.01 to about 10 weight percent, and in some aspects from about 0.2 to about 10 weight percent, on a basis of total weight of the topical formulation.

Non-limiting examples of useful antioxidants include butylated hydroxyanisole, butylated hydroxytoluene, edetate disodium or mixtures of two or more thereof

The amount of antioxidant in the topical formulation can range from about 0.01 to about 1 weight percent, and in some aspects from about 0.01 to about 0.1 weight percent, on a basis of total weight of the topical formulation.

Non-limiting examples of useful humectants include ethylene glycol, glycerin, sorbitol or mixtures of two or more thereof. The amount of humectant in the topical formulation can range from about 1 to about 30 weight percent, and in some aspects from about 2 to about 20 weight percent, on a basis of total weight of the topical formulations.

Non-limiting examples of useful emulsifiers include acrylic acid polymners (such as carbomer brand thickeners e.g. Carbomer 934P, manufactured by Voveon, inc.), polyoxyethylene-10-stearyl ether, polyoxyethylene-20-stearyl ether, cetostearyl alcohol, cetyl alcohol, cholesterol, diglycol stearate, glyceryl monostearate, glyceryl stearate, polygeyceryl-3-oleate, hydroxypropyl cellulose, hydroxypropylmethyl cellulose, lanolin, polyoxyethylene lauryl ether, methyl cellulose, polyoxyethylene stearate, polysorbate, propylene glycol monostearate, sorbitan esters, stearic acid or mixtures of two or more thereof.

The amount of emulsifier in the topical formulation can range from about 1 to about 40 weight percent, and in some aspects from about 5 to about 30 weight percent, on a basis of total weight of the topical formulation.

The gel formulations of the present disclosure comprise one or more gelling agents. Non-limiting examples of useful gelling agents includecarboxylic acid polymers including acrylic acid plymers crossl linkcd with cross links such as allyl ethers of sucrose (e.g. carbomer brand thickeners), cetostearyl alcohol, hydroxymethyl cellulose, polyoxyethylene-polyoxypropylene copolymer, sodium carboxymethylcellulose, polyvinyl pyrrolidone, or mixtures of two or more thereof.

The amount of gelling agent in the topical gel formulation can range from about 0.1 to about 10 weight percent, and in some aspects from about 0.1 to about 1 weight percent, on a basis of total weight of the topical formulation.

The gel formulations of the present disclosure can further comprise one or more alkalinizers, for example sodium hydroxide, in amount of less than about 2 weight percent as activators ofgelling. The formulations can contain one or more additional excipients well known in the art, for example water and a thickening agent such as colloidal silicon dioxide.

The formulations of the present disclosure can be administered in combination with one or more other antiviral or other agents useful in treating or preventing infection with HIV or in inhibiting transmission ofHIV, in combination with a pharmaceutically acceptable carrier. In one form of the method, the subject is under treatment with an antiretroviral agent. In some aspects, the method comprises coadministration of an antiretroviral agent, and particularly an agent used for the treatment of HIV infection such as Zidovudine (AZT), Abacavir, Emtricitabine (FTC), Lamivudine (3TC), Didanosine (ddl), Stavudine (d4T), Zalcitabine (ddC), Nevirapine, Efavirenz, Delavirdine, Tenofovir, Enfuvirtide (T20), Maraviroc (CCR5), Lopinavir, Atazanavir, Fosamprenvir, Amprenavir, Saquinavir, Indinavir, Nelfinavir, Raltegravir, and Elvitegravir.

One or more, preferably one to four, antiviral agents useful in anti- H IV-1 therapy may be used in combination with at least one (i.e., 1 -4, preferably 1) anti-Env antibody in a formulation of the present disclosure. The antiviral agent or agents may be combined with the anti-Env antibody in a single dosage form, or the anti-Env antibody and the antiviral agent or agents may be administered simultaneously or sequentially as separate dosage forms. For example, the anti-Env antibody formulation can be used in a vaginal ring device or to coat the outside of a condom to prevent transmission of HIV to a non-infected sexual partner while the HIV-infected sexual partner undergoes treatment with systemic antiviral therapy. The antiviral agents contemplated for use in combination with the anti-Env antibody formulations of the present disclosure comprise nucleoside and nucleotide reverse transcriptase inhibitors, non-nucleoside reverse transcriptase inhibitors, protease inhibitors and other antiviral drugs listed below not falling within these classifications. In particular, the combinations known as HAART are contemplated for use in combination with the anti-Env antibody formulations of this disclosure. [00169] The term "nucleoside and nucleotide reverse transcriptase inhibitors" ("NRTI" s) as used herein means nucleosides and nucleotides and analogues thereof that inhibit the activity of HIV-1 reverse transcriptase, the enzyme which catalyzes the conversion of viral genomic HIV-1 RNA into proviral HIV-1 DNA. Typical suitable NRTIs include zidovudine (AZT) available under the RETROVIR tradename from Glaxo-Wellcome Inc., Research Triangle, NC 27709; didanosine (ddl) available under the VIDEX tradename from Bristol- Myers Squibb Co., Princeton, NJ 08543; zalcitabine (ddC) available under the HMD tradename from Roche Pharmaceuticals, Nutley, NJ 071 10; stavudine (d4T) available under the ZERIT trademark from Bristol-Myers Squibb Co., Princeton, NJ 08543; lamivudine (3TC) available under the EPIVIR tradename from Glaxo-Smith Kline Triangle, NC 27709; abacavir (1592U89) disclosed in WO96/30025 and available under the ZIAGEN trademark from Glaxo- Wellcome Research Triangle, NC 27709; adefovir dipivoxil [bis(POM)-PMEA] available under the PREVON tradename from Gilead Sciences, Foster City, CA 94404; lobucavir (BMS-180194), a nucleoside reverse transcriptase inhibitor disclosed in EP-0358154 and EP- 0736533 and under development by Bristol-Myers Squibb, Princeton, NJ 08543; BCH-10652, a reverse transcriptase inhibitor (in the form of a racemic mixture of BCH-10618 and BCH-10619) under development by Biochem Pharma, Laval, Quebec H7V, 4A7, Canada; emitricitabine [(-)-FTC] licensed from Emory University under Emory Univ. U.S. Patent No. 5,814,639 and available from Gilead under the trade name Emtrivia™; beta-L-FD4 (also called beta-L-D4C and named beta- L-2', 3'-dicleoxy-5-fluoro-cytidene) licensed by Yale University to Vion Pharmaceuticals, New Haven CT 0651 1 ; DAPD, the purine nucleoside, (-)- beta-D-2,6,-diamino-purine dioxolane disclosed in EP 0656778 and licensed by Emory University and the University of Georgia to Triangle Pharmaceuticals, Durham, NC 27707; and lodenosine (FddA), 9-(2,3-dideoxy- 2-fluoro-b-D-threo-pentofuratiosyl)adenine, an acid stable purine-based reverse transcriptase inhibitor discovered by the NIH and under development by U.S. Bioscience Inc., West Conshohoken, PA 19428. [00171] The term "non-nucleoside reverse transcriptase inhibitors" ("NNRTI1 1 S) as used herein means non-nucleosides that inhibit the activity of HIV-1 reverse transcriptase.

Typical suitable NNRTIs include nevirapine (BI-RG-587) available under the VIRAMUNE tradename from Boehringer Ingelheim, the manufacturer for Roxane Laboratories, Columbus, OH 43216; delaviradine (BHAP, U-90152) available under the RESCRIPTOR tradename from Pharmacia & Upjohn Co., Bridgewater NJ 08807; efavirenz (DMP-266) a benzoxazin-2-one disclosed in WO94/03440 and available under the SUSTIVA tradename from Bristol Myers Squibb in the US and Merck in Europe; PNU-142721 , a furopyridine-thio- pyrimide under development by Pharmacia and Upjohn, Bridgewater NJ 08807; AG-1549 (formerly Shionogi # S-1 153); 5-(3,5-dichlorophenyl)- thio-4- isopropyl-1 -(4-pyridyl)methyl-IH-imidazol-2-ylmethyl carbonate disclosed in WO 96/10019 and under clinical development by Agouron Pharmaceuticals, Inc., LaJolla CA 92037-1020; MKC-442 (1 -(ethoxymethyl)-5-(1-methylethyl)-6-(phenylmethyl)-(2,4(1H,3H)- pyrimidinedione) discovered by Mitsubishi Chemical Co. and under development by Triangle Pharmaceuticals, Durham, NC 27707; (+)- calanolide A (NSC-675451) and B, coumarin derivatives disclosed in NIH U.S. Patent No. 5,489,697, licensed to Med Chem Research, which is co- developing (+) calanolide A with Vita-Invest as an orally administrable product; and etravirine (TMC-125, Intelence) marketed by Tibotec. [00173] The term "protease inhibitor" ("PI") as used herein means inhibitors of the HIV-1 protease, an enzyme required for the proteolytic cleavage of viral polyprotein precursors (e.g., viral GAG and GAG Pol polyproteins), into the individual functional proteins found in infectious HIV-1 . HIV protease inhibitors include compounds having a peptidomimetic structure, high molecular weight (7600 daltons) and substantial peptide character, e.g. CRIXIVAN (available from Merck) as well as nonpeptide protease inhibitors e.g., VIRACEPT (available from Agouron).

Typical suitable Pis include saquinavir (Ro 31 -8959) available in hard gel capsules under the INVIRASE tradename and as soft gel capsules under the FORTOVASE tradename from Roche Pharmaceuticals, Nutley, NJ 071 10- 1 199; ritonavir (ABT-538) available under the NORVIR tradename from Abbott Laboratories, Abbott Park, IL 60064; indinavir (MK-639) available under the CRIXIVAN tradename from Merck & Co., Inc., West Point, PA 19486-0004; nelfnavir (AG-1343) available under the VIRACEPT tradename from Agouron Pharmaceuticals, Inc., LaJolla CA 92037-1020; amprenavir (141 W94), tradename AGENERASE, a non-peptide protease inhibitor under development by Vertex Pharmaceuticals, Inc., Cambridge, MA 02139-421 1 and available from Glaxo-Wellcome, Research Triangle, NC under an expanded access program; lasinavir (BMS-234475) available from Bristol- Myers Squibb, Princeton, NJ 08543 (originally discovered by Novartis, Basel, Switzerland (CGP-61755); DMP-450, a cyclic urea discovered by Dupont and under development by Triangle Pharmaceuticals; BMS-2322623, an azapeptide under development by Bristol-Myers Squibb, Princeton, NJ 08543, as a 2nd-generation HIV-1 PI; ABT-378 under development by Abbott, Abbott Park, IL 60064; AG-1549 an orally active imidazole carbamate discovered by Shionogi (Shionogi #S-1 153) and under development by Agouron Pharmaceuticals, Inc., LaJolla CA 92037-1020; atazanavir; tipranavir; and darunavir.

Other antiviral agents include CXCR4 antagonists, enfuvirtide, hydroxyurea, ribavirin, IL-2, IL-12, pentafuside and Yissum Project No. 1 1607. Hydroxyurea (Droxia), a ribonucleoside triphosphate reductase inhibitor, the enzyme involved in the activation of T-cells, was discovered at the NCI and is under development by Bristol-Myers Squibb; in preclinical studies, it was shown to have a synergistic effect on the activity of didanosine and has been studied with stavudine. IL-2 is disclosed in Ajinomoto EP-0142268 , Takeda EP- 0176299, and Chiron U. S. Patent Nos. RE 33653, 4530787, 4569790, 4604377, 4748234, 4752585, and 4949314, and is available under the PROLEUKIN (aldesleukin) tradename from Chiron Corp., Emeryville, CA 94608-2997 as a lyophilized powder for IV infusion or sc administration upon reconstitution and dilution with water; a dose of about 1 to about 20 million ILJ/day, se is preferred; a dose of about 15 million lU/day, sc is more preferred. IL-12 is disclosed in WO96/25171 and is available from Roche Pharmaceuticals, Nutley, NJ 071 10-1 199 and American Home Products, Madison, NJ 07940; a dose of about 0.5 microgram/kg/day to about 10 microgram/kg/day, sc is preferred. Enfuvirtide (DP-178, T-20) a 36-amino acid synthetic peptide, is disclosed in U.S. Patent No.5,464, 933 licensed from Duke University to Trimeris which developed enfuvirtide in collaboration with Duke University and Roche; enfuvirtide acts by inhibiting fusion of HIV-1 to target membranes. Enfuvirtide (3-100 mg /day) is given as a continuous sc infusion or injection together with efavirenz and 2 Pi's to HIV-1 positive patients refractory to a triple combination therapy; use of 100 mg/day is preferred. Yissum Project No. 1 1607, a synthetic protein based on the HIV -1 Vif protein, is under preclinical development by Yissum Research Development Co., Jerusalem 91042, Israel. Ribavirin, I-β-D-ribofuranosyl-1H-1 ,2,4-triazole-3-carboxamide, is available from ICN Pharmaceuticals, Inc., Costa Mesa, CA; its manufacture and formulation are described in U.S. Patent No. 4,21 1 ,771 ; the integrase inhibitor raltegravir available from Merck under the tradename Isentress™; elvitegravir an intergrase inhibitor under development by Gilead Sciences; the HIV-1 Gag maturation inhibitor berivimat under development (Phase lib) by Panacos Pharmaceuticals. [00176] The term "anti-HIV-1 therapy" as used herein means any anti-HIV-1 drug found useful for treating HIV-1 infections in man alone, or as part of multidrug combination therapies, especially the HAART triple and quadruple combination therapies. Typical suitable known anti-HIV-1 therapies include, but are not limited to multidrug combination therapies such as (i) at least three anti-HIV-1 drugs selected from two NRTIs, one PI, a second PI, and one NNRTI; and (ii) at least two anti-HIV-1 drugs selected from NNRTIs and Pis. Typical suitable HAART-multidrug combination therapies include: [00177] (a) triple combination therapies such as two NRTIs and one PI; or (b) two NRTIs and one NNRTI; and (c) quadruple combination therapies such as two NRTIs, one PI and a second PI or one NNRTT. Tn treatment of naive patients, it is preferred to start anti-HIV-1 treatment with the triple combination therapy; the use of two NRTIs and one NNRTI or two NRTIs and one PI is preferred if there is intolerance to NNRTI. Drug compliance is essential. The CD4+ and HIV-1 -RNA plasma levels should be monitored every 3-6 months. Should viral load plateau, a fourth drug, e.g., one PI, one NNRTI or integrase inhibitor could be added.

The present disclosure also provides the use of a composition of the present disclosure for the manufacture of a medicament for the treatment and/or prevention of HIV transmission.

The present disclosure also provides a method of preparing a composition for inhibiting transmission of HIV comprising immunizing an animal with a HIV viral envelope (Env) protein or a fragment thereof, and obtaining hyperimmune colostrum from the immunized animal.

The present disclosure also provides a composition for inhibiting transmission of H IV prepared by the method comprising immunizing an animal with a HIV viral envelope (Env) protein or a fragment thereof, and obtaining hyperimmune milk from the immunized animal.

The present disclosure also provides a method of inhibiting transmission of HIV comprising:
forming hyperimmune colostrum or hyperimmune milk by immunizing cows; and
administering the hyperimmune colostrum or hyperimmune milk to a subject, wherein the step of immunizing cows to produce hyperimmune colostrum or hyperimmune milk comprises vaccination with a human immunodeficiency virus (HIV) viral envelope (Env) protein or a fragment thereof. The Env protein or fragment thereof may be any HIV Env protein, however preferably the Env protein is gp140. Also provided is a method for inhibiting transmission of HIV comprising administering polyclonal antibodies or fragments thereof capable of binding to a human immunodeficiency virus (HIV) viral envelope (Env) protein or a fragment thereof to a subject. The Env protein or fragment thereof may be any HIV Env protein, however preferably the Env protein is gp140.

In one aspect, the Env protein or fragment thereof is a gp140 oligomer. The oligomer may comprise gp140 trimers, dimers and monomers. The Oligomers may be purified from transduced HeLa and 293 cell supernatant, for example by lentil lectin affinity chromatography and gel filtration. The Env protein or fragment thereof may be a H IV clade A, clade B or clade C strain viral envelope (Env) protein or fragment thereof.

In one aspect, the polyclonal antibodies or fragments thereof are capable of binding to a Env protein from a heterologous clade of HIV or a heterologous strain of H IV.

The antibody, or fragment thereof, or functional equivalent thereof may be produced by immunization of an animal with a HIV viral envelope (Env) protein or a fragment thereof. The animal may be immunized with gp140, recombinant gp140 or oligomeric 140. The recombinant gp140 may not be derived from virion culture. The animal may be immunized with a HIV viral envelope (Env) protein or a fragment thereof and an adjuvant. In one aspect, the adjuvant is a water in oil emulsion.

The antibody, or fragment thereof, or functional equivalent thereof may be present in or obtained from an avian egg, or present in or obtained from hyperimmune colostrum or hyperimmune milk of an animal. The animal may be a cow.

The composition may be formulated for topical administration, and in certain aspects the composition is formulated for vaginal or rectal administration. The composition may be formulated as a gel, or formulated as a topical cream, ointment, lotion or foam formulation.

In one aspect of the method, the ligand is an antibody, or fragment or derivative thereof. The antibodies or fragment or derivative thereof may be polyclonal immunoglobulins or chimeric antibodies or dendrimer presented immunoactive fragments or immunoactive fragments such as F(ab) and F(ab)2 fragments or recombinant immunoactive fragments, or affinity purified immunoglobulins or immunoactive fragments thereof.

In one form of the composition the antibody or fragment thereof or derivative thereof is produced by immunization of an animal with a microbe or a microbial product. Polyclonal antibodies capable of binding to a microbe or microbial product may be obtained by the immunization of an animal, and obtaining the antibodies via a bodily fluid, such as blood, a secretion of a gland or cell, egg, milk or colostrum. The methods, compositions and devices of this disclosure can be adapted generally to release active agent in a time sensitive manner that best corresponds to the timing of sexual activity. When topically applied as a lotion or gel, the compositions are preferably applied immediately prior to sexual activity. Other modes of application, such as devices and suppositories, can be designed to release active agent over a prolonged period of time, at a predetermined rate, depending upon the needs of the consumer.

In certain aspects of the present disclosure, the goal of the formulations of the present disclosure is to reduce the HIV-1 -RNA viral load below the detectable limit so that infection or transmission of infection is slowed, prevented or inhibited. The "detectable limit of HIV-1 -RNA" in the context of the present disclosure means that there are fewer than about 200 to fewer than about 50 copies of HIV-1 -RNA per ml of plasma of the patient as measured by quantitative, multi-cycle reverse transcriptase PCR methodology. HTV-1 -RNA is preferably measured in the present disclosure by the methodology of Amplicor -1 Monitor 1.5 (available from Roche Diagnostics) or of Nuclisens HIV-1 QT -1. In certain aspects, the formulations of the disclosure are useful to protect not only against sexual transmission of HIV, but also to prevent infection of a baby during passage through the birth canal. Thus the vaginal administration can take place prior to sexual intercourse, during sexual intercourse, immediately prior to childbirth or during childbirth. Such topical dosage forms may be particularly useful when applied to a newborn baby of an HIV-infected mother.

The present invention will now be more fully described by reference to the following non-limiting Examples.

### EXAMPLES

### EXAMPLE 1 : Production of Hyperimmune Colostrum containing polyclonal anti-Env antibodies

### Step 1 - Production of vaccine for dairy cattle

The procedures for preparing antigen reported in Pub. No. WO/2004/078209 International Application No. PCT/AU2004/000277 were used. Step 2 - The procedures for preparing antibodies from vaccinated cattle reported in Pub. No. WO/2004/078209 International
Application No. PCT/AU2004/000277 were used.

### EXAMPLE 2: Production of polyclonal antibodies binding to HIV Env, and demonstration of neutralization

Soluble Env gp140 oligomers have been prepared from clade A, B, and C HIV-1 strains from HeLa and/or 293T cells and purified by lentil lectin affinity and gel filtration chromatography.

Four cows (two pregnant in second semester and two initially non-pregnant) were vaccinated with 100 g of purified HIV-1 Env gp140 oligomer formulated with Montanide adjuvant. Two groups of two cows (one pregnant and one nonpregnant) were vaccinated with either clade B (AD8) only or with equal amounts (33.33Mg) of clade A, B and C Env gp140 (UG8, AD8 and MW) (referred to as 'trimix'). All four cows received at least three vaccinations whereas the last vaccination was given four weeks before giving birth. All four cows seroconverted within nine weeks. Reciprocal endpoint serum IgG titers were up to 1 x10^{2 5}for pregnant cows and up to 1 x10⁵ for non-pregnant cows determined by a new established anti-bovine IgG HIV-1 Env gp140 specific ELISA. The expected low serum IgG titer in pregnant cows was explained by the pumping of serum IgG antibodies into the colostrum approximately four weeks before giving birth.

HIV-immune bovine colostrum was collected and pasteurised postpartum from all cows with pregnancy vaccination resulting in relatively low responses with reciprocal IgG titers of < 10² (clade B vaccinated) and 1 x 10^{3 5} (trimix- vaccinated). Reciprocal colostrum IgG titer for cows vaccinated before pregnancy was 10⁵ (clade B vaccinated) and 10^{4 5} (trimix vaccinated). Western blot analysis confirmed that colostrum IgG of all four cows was specific against HIV-1 Env gp140. Unfractionated colostrum was tested for neutralising activity in a HIV-1 Env-pseudotyped reporter virus assay. Clade A E, clade B and clade C pseudotype viruses including the NIH reference panel for clade B and C viruses were tested (total n=27) and compared with non-immune bovine colostrum that already has intrinsic infection-blocking activity due to lactoferrin and other bioactive peptides. Unfractionated colostrum from the trimix cow vaccinated during pregnancy showed high neutralisation of up to 50% for all B clade pseudoviruses (n=15) as well as for the majority of C clade (n=1 1) and clade A E (n=1) pseudoviruses at a dilution of 1 :16. The first clade B vaccinated cow was a low responder but both cows vaccinated before pregnancy and having their calves recently responded well. Up to this time, broad neutralisation was observed for the clade B vaccinated cow that showed 50- 80% neutralisation for B clade (n=12) and clade C pseudoviruses (n=9) (1 :16 dilution) (Table1). IgG Abs from the first pair of cows was purified from the colostrum and neutralising activity was retained for purified IgG with up to 50% neutralisation for the trimix- IgG compared to non immune IgG at 500 g/ml.

Results of the neutralisation profile of two HIV Env gp140 hyperimmune bovine colostrum samples against pseudoviruses of different clades are demonstrated in Table 1.

These results strongly support this method of raising high levels of neutralising antibodies. Table 1

| | **Clade** | **Neutralisation (%) (1:16)** | | |
|---|---|---|---|---|
| Animal number | | **6055** | **7004** | **non-immune** |
| Reciprocal IgG titer in H2BC | | 103.5 | 105 | *!* |
| gp140 specificity | | + | + | *!* |
| Immunisation / Ag | | Trimix AE/B/C | clade B | *!* |

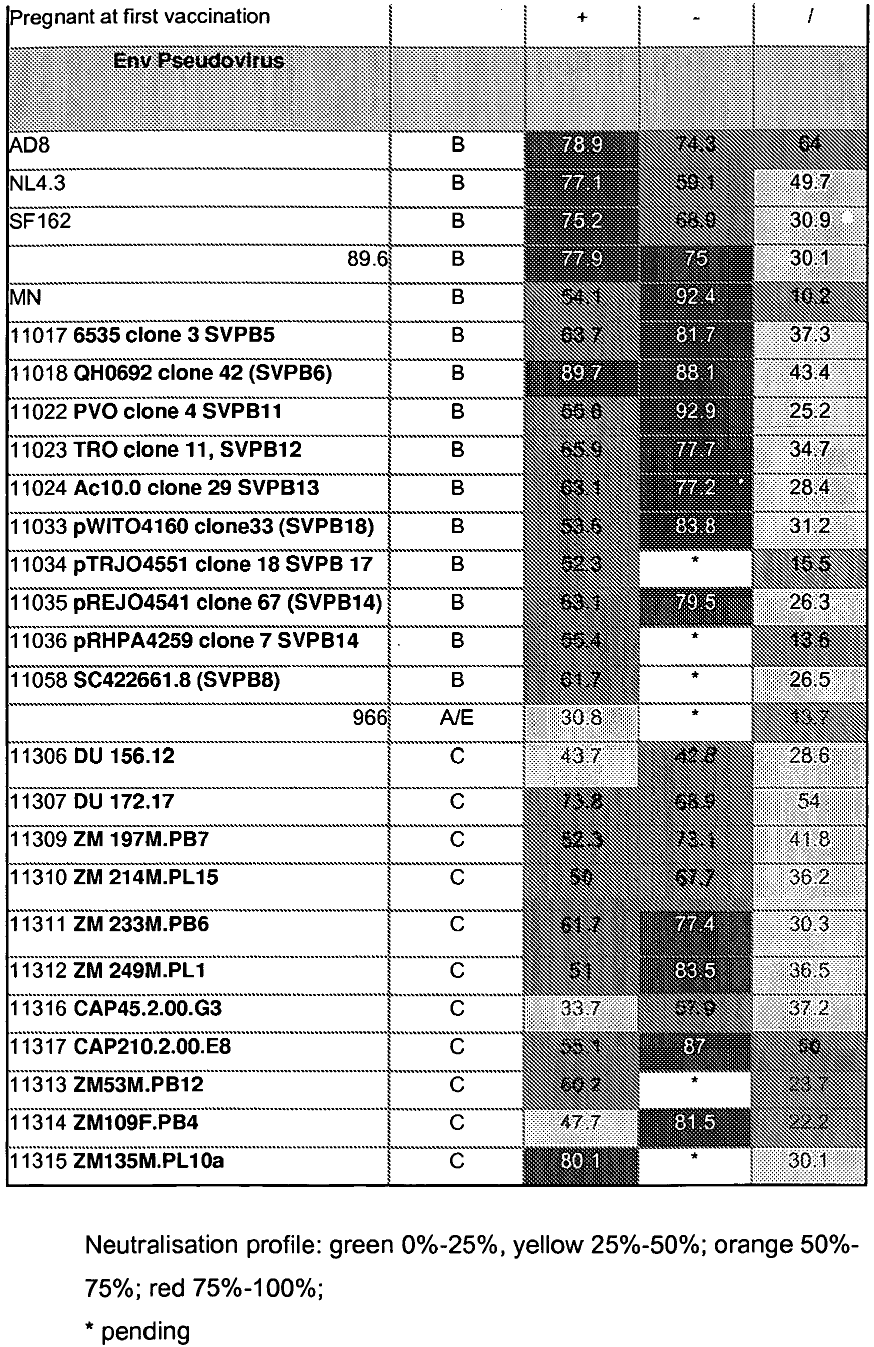

### EXAMPLE 3: Polyclonal neutralising antibodies to HIV-1 Env from bovine colostrum.

Twelve BSE-free pregnant cows housed in an approved quarantine farm in Victoria are vaccinated with 100 g of an eqimolar mix of four HIV-1 Env gp140 oligomers:
1) SC35 clade B pre-seroconversion strain Env gp140, these adopt an open configuration and prominently displays important neutralisation epitopes;
2) ADA primary R5-tropic clade B Env gp140;
3) 966 clade A/E Env gp140; and
4) MW clade C Env gp140.

These Env are formulated with adjuvant (Montanide) and administered twice before pregnancy and at least twice at 3-week intervals during the second trimester of pregnancy by a registered veterinarian.

An Env ELISA assay and Western blotting are used to monitor the levels of Env-specific IgG in regular blood samples taken during the vaccination and pregnancy and vaccination is continued until high titres of IgG are detected. Immediately following calving, the first colostrum is collected by a registered veterinarian and calves are given their essential colostrum, leaving around one litre of colostrum per cow.

Following storage of200mls of whole colostrum, the remainder is fractionated and purified using methods to yield 1 kg of pure freeze dried antibodies. Whole colostrum and purified antibodies are assessed for breadth and titre of neutralizing antibody activity using a Env-pseudotyped reporter virus assay. Typical target cells are tested in these assays, as well as primary cells. HIV neutralisation is also confirmed in the PBMC spreading infection system. Neutralisation assays for SIV to assess if this robust challenge model can be used for primate studies are also examined. Importantly, antibodies are titrated into pooled seminal plasma that is by-product from IVF clinical procedures, and into vaginal washings collected at various stages of the menstrual cycle and tested for neutralising activity. Usually the pH in the vagina is acid (between pH 4 and 5) but in the presence of semen the pH is increased to a neutral (pH 7) level. The activity of bovine colostrum antibodies are tested across this pH range.

### EXAMPLE 4: Mechanisms ofHIV inhibition by bovine colostrum IgG in viral transcytosis and neutralisation assays in vitro and in a cervical explant infection model.

One major path by which HIV circumvents the host defence mechanisms is the transport of the virus within a protective vesicle across the interior of epithelial cells that face the inside of the vagina (known as transcytosis) without infecting these cells. Anti-transcytosis activity of bovine polyclonal antibodies are assessed in Hec 1 -B cells using an EVOM2 Epithelia tissue voltohmmeter in a transcytosis assay. Whole colostrum, in addition to purified IgA and IgG are examined for anti-transcytosis activity. Cervical tissue is obtained, and the penetration of fluorescently labelled bovine IgG into the epithelial layers of the ectocervix, endocervix and columnar epithelium of the the vagina is examined. HIV virion labelled with fluorescent Vpr is added to track the movement ofHIV on and through these tissues in the explant culture model to observe virolysis or entrapment.

### EXAMPLE 5: Formulation of colostrum neutralizing antibodies into a microbicide gel and testing gel safety and efficacy in rabbits.

The most potent HIV-1 polyclonal bovine anti-Env antibodies are tested and pooled and formulate into various water-based buffering gels. Several formulations of anti-Env Ab are prepared, including formulations that add back lactoferrin as a protein excipient, because it also has potent neutralising activity, and a cascin and calcium carbonate formulation to test the possibility of retaining IgG binding activity after passage through the stomach and alimentary system for an oral delivered rectal microbicide.

The activity of antibodies when coformulated into different existing gels, such as glycol based K-Y lubricant gel, or the dendrimer microbicide gel, Vivagel, developed by Starpharma. Formulations that retain or enhance the breadth and potency of HIV neutralisation are tested for activity under neutral as well as acid pH conditions in vitro and their stability. The stability, biodistribution and reactogenicity/inflammation induced by the bovine antibodies are tested in rabbit toxicology studies. Biodistribution of bovine IgG are examined by histopathology, immunohistocehmistry, dermal observation of local inflammatory responses, immunogenicity by antibody and cellular responses to bovine IgG, and by Bioplex bead arrays or cytokine ELISA and EliSpot assay. Body weight, ophthalmology, ECG, clinical chemistry, haematology, urinalysis, organ weights and bone marrow and blood smears are tested for any abnormality. Control animals are given a placebo gel. Following cell toxicity studies, colostrum and purified Abs are co-cultivated with bacteria common in the vagina e.g. Lactobacillus acidophilus to assess the effect of colostrum Ab on the beneficial normal flora. Prior to primate challenge studies, the stability and activity of the formulation in the vaginal environment is tested at different time points following application to rabbits by recovering antibodies by saline washing.

### EXAMPLE 6: Formulations

The following are formulated according to standard methods based on the following lists of ingredients. 'Hyperimmune colostrum' is hyperimmune colostrum containing antibodies to Env.

### Formulation 1

A vaginal cream formulation is prepared by mixing the components listed in Table 2 below. For each application, 1 -4 grams of the cream are vaginally administered with a suitable applicator such as a syringe.

**Table 2**

| Component | Weight Percent |
|---|---|
| Hyperimmune colostrum | 10-40 |
| Cetyl esters wax | 1-15 |
| Cetyl alcohol | 2-5 |
| White wax | 5-20 |
| Glyceryl monostearate | 10-30 |
| Propylene glycol monostearate | 10-15 |
| Methyl stearate | 5-90 |
| Benzyl alcohol | 3-10 |
| Sodium lauryl sulfate | 0.5-2.5 |
| Glycerin | 5-30 |
| Mineral oil | 0.1-95 |

### Formulation 2

A vaginal cream formulation is prepared by mixing the components listed in Table 3 below. For each application, 1 -4 grams of the cream are vaginally administered with a suitable applicator such as a syringe.

**Table 3**

| Component | Weight Percent |
|---|---|
| Hyperimmune colostrum | 10-40 |
| edetate disodium | 0.01-0.10 |
| glyceryl monoisostearate | 0.5-10 |
| methyl paraben | 0.18-0.20 |
| mineral oil | 0.1-95 |
| polyglyceryl-3-oleate | 2-3.5 |
| propylene glycol | 5-15 |
| propyl paraben | 0.02-0.10 |
| colloidal silicon dioxide | 1-5 |
| sorbitol solution | 2-18 |
| purified water | 10-20 |
| microcrystalline wax | 2-20 |

### Formulation 3

A vaginal gel formulation is prepared by mixing the components listed in Table 4 below. For each application, 4 grams of the gel are vaginally administered with a suitable applicator such as a syringe.

**Table 4**

| Component | Weight Percent |
|---|---|
| Hyperimmune colostrum | 10-40 |
| Carbomer 934P | 0.1-0.5 |
| Edetate disodium | 0.01-0.10 |
| Methyl paraben | 0.18-0.20 |
| Propyl paraben | 0.02-0.10 |
| Propylene glycol | 5-15 |
| Sodium hydroxide | 0.01-0.05 |

### Formulation 4

A rectal foam formulation is prepared by mixing the components listed in Table 5 below and inert propellants isobutene and propane. The foam is supplied in a aerosol container with a rectal applicator. For each application, 900 milligrams of the foam are rectally administered using the applicator.

**Table 5**

| Component | Weight Percent |
|---|---|
| Hyperimmune colostrum | 10-40 |
| Propylene glycol | 5-15 |
| Emulsifying wax | 10-15 |
| Polyoxyethylene-10-stearyl ether | 0.1-0.5 |
| Cetyl alcohol | 2-5 |
| Methyl paraben | 0.18-0.20 |
| Propyl paraben | 0.02-0.10 |
| Triethanolamine | 2-4 |
| Purified water | 10-30 |

### EXAMPLE 7: Antigen production, Purification and Vaccination of Cows

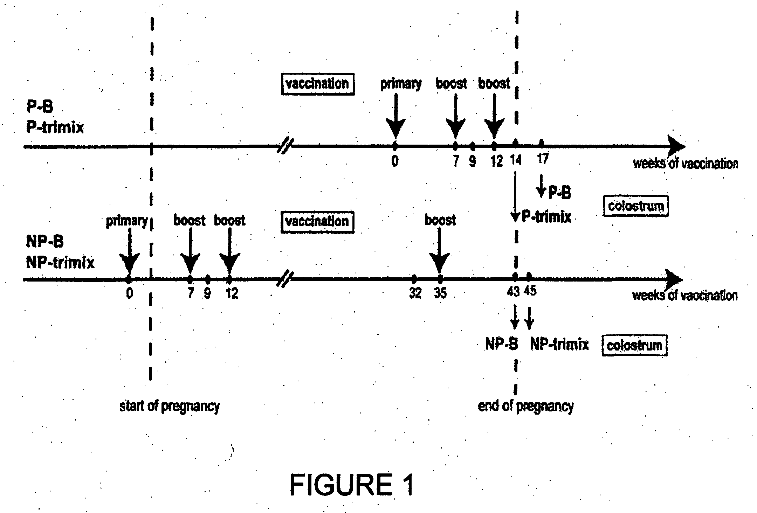

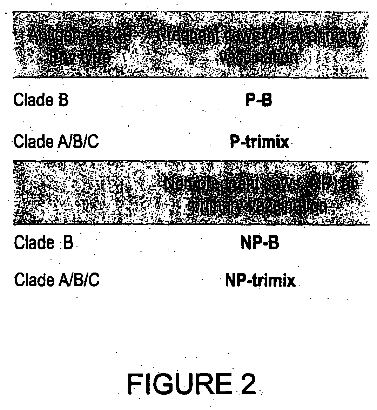

Soluble trimeric clade A (92UG8037.8; UG8), clade B (AD8) and clade C (93MW965.26; MW) HIV-1 Env gp140 were purified by lentil lectin affinity chromatography and gel filtration and 100ug of gp140 in a proprietary adjuvant was used for three intramuscular vaccinations of 4 cows; 2 cows after conception (p) and two cows before conception (NP) according to the vaccination schedule shown in Figures 1 and 2.

### EXAMPLE 8: IgG from serum and colostrum binds to gp140 Env of clade A, B and C

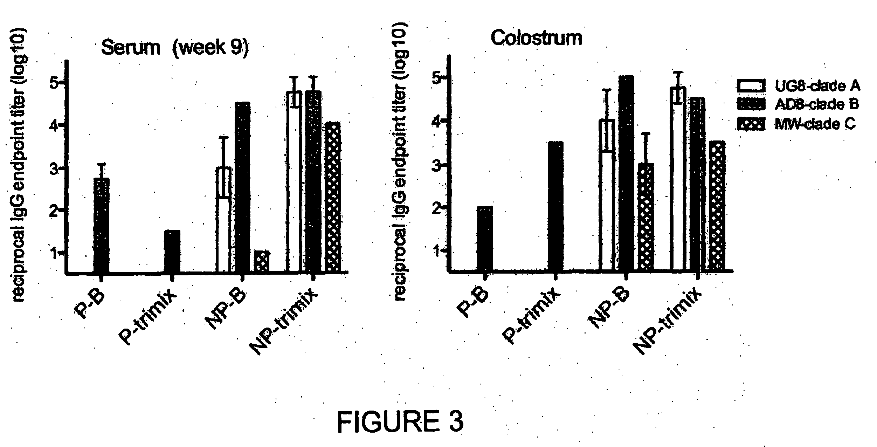

Figure 3 shows the Env gp-140-specific IgG itires in serum 9 weeks following primary vaccination and colostrum determined by direct ELISA against gp140 Env of clade A (UG8), clade B (AD8) and clade C (MW). Reciprocal endpoint titres were determined using a 2 times OD cut-off based on pre-bleed samples or non-immune colostrum respectively. These results demonstrate IgG from serum and colostrum binds to gp140 Env of clade A, R and C. The results also demonstrate IgG from non-pregnant cows have broad binding activity to gp140 of clades A, B and C.

### EXAMPLE 9: Purified colostrum IgG from non-pregnant cows retains binding to gp140 Env

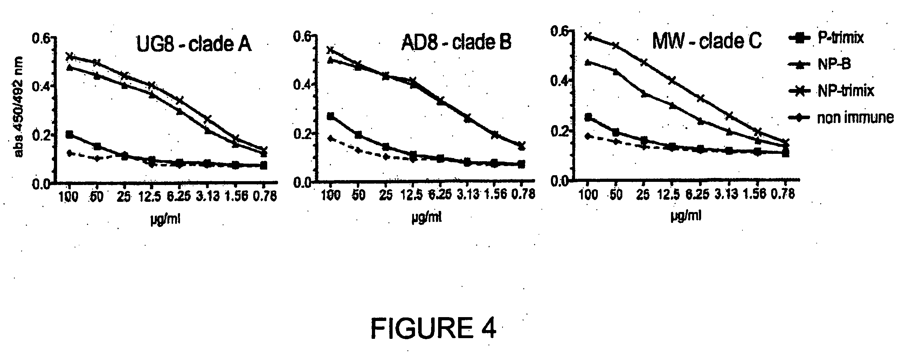

Figure 4 shows the specific binding activity of purified colostrum IgG determined by direct ELISA against clade A (UG8), clade B (AD8) and clade C (MW) and absorbance (abs) measurement at 450nm. These results demonstrate purified IgG from non-pregnant cows retains binding to gp140. The results also demonstrate purified IgG from non-pregnant cows retains broad binding activity to gp140 of clades A, B and C. The results also demonstrate cross-clade (heterologous) binding, with colostrum from nonpregnant cows vaccinated with clade B soluble Env gp140 oligomers binding gp140 of clades A B and C.

### EXAMPLE 10: Bovine IgG blocks binding of monoclonal Ab b12 to CD4 binding site of gp140

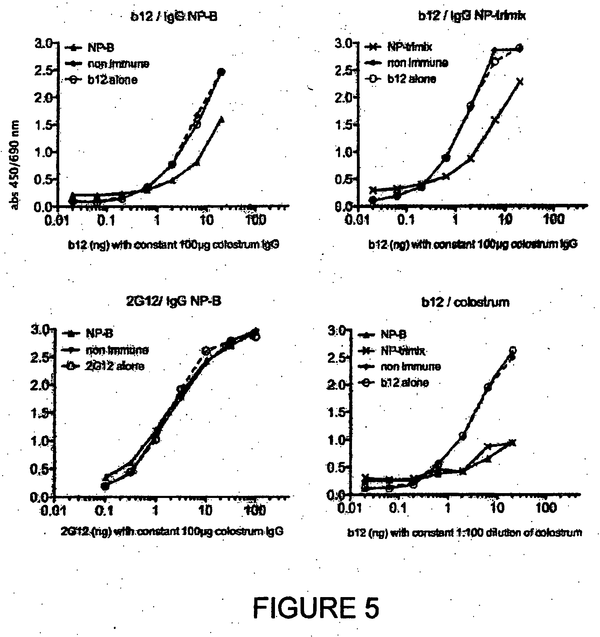

Several broadly neutralizing human monoclonal antibodies (MAbs) have been derived from infected individuals, including immunoglobulin G1 (lgG1) b12 and 2G12. Among the most potent, the well-known b12 monoclonal antibody (Ab b12) occludes the site of CD4 binding on gp120 (which forms part of gp140) and prevents virus attachment to CD4 on target cells and is able to neutralize primary HIV-1 isolates. 2G12 recognizes a cluster of high mannose glycans on the viral envelope glycoprotein gp120. An understanding of the specificity of b12 binding, neutralization, and protection should aid in the development of immunogens that induce neutralizing antibodies of a similar specificity Figure 5 shows bovine colostrum IgG competes with human neutralizing mAb b12 for binding at gp140 CD4 binding site. Competition ELISAs were performed by titrating b12 and 2G 12 in a constant background of 100ug IgG or a 1 : 100 dilution of whole colostrum. The ability of b12 and 2G12 to bind to AD8 (clade B gp140) in the presence or absence of colostrum IgG was detected by anti-human IgG HRP conjugated antibody. The results demonstrate bovine IgG blocks binding of the potent b12 antibody to the CD4 binding site of gp140 Env. The results also demonstrate bovine IgG from nonpregnant cows blocks binding of the potent b12 antibody to the CD4 binding site of gp140 Env.

### EXAMPLE 11: Colostrum from pregnant cows vaccinated with clade A/B/C gp140 and non-pregnant cows vaccinated with clade B gp140 have broad neutralizing activity.

Table 6 shows the neutralization profile of whole colostrum. Numbers represent percent neutralization for a 1 :16 dilution against the indicated EGFP Env-pseudotyped viruses including common lab strains and the NIH clade B and C reference panel (ARRP #1 1227, #1 1326) in CF2 cells. Data shown is a representative experiment from two independent experiments. The results demonstrate colostrum from pregnant cows vaccinated with clade A/B/C gp140 and non-pregnant cows vaccinated with clade B gp140 have broad neutralizing activity. In particular, this neutralization is cross-clade (heterologous) neutralization, with colostrum from non-pregnant cows vaccinated with clade B soluble Env gp140 oligomers neutralizing HIV of clades A B and C. Furthermore, colostrum from pregnant cows vaccinated with trimix soluble Env gp140 oligomers neutralizing HIV of clades A B and C. The results also show that non-immune colostrum has neutralizing activity.

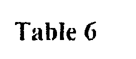

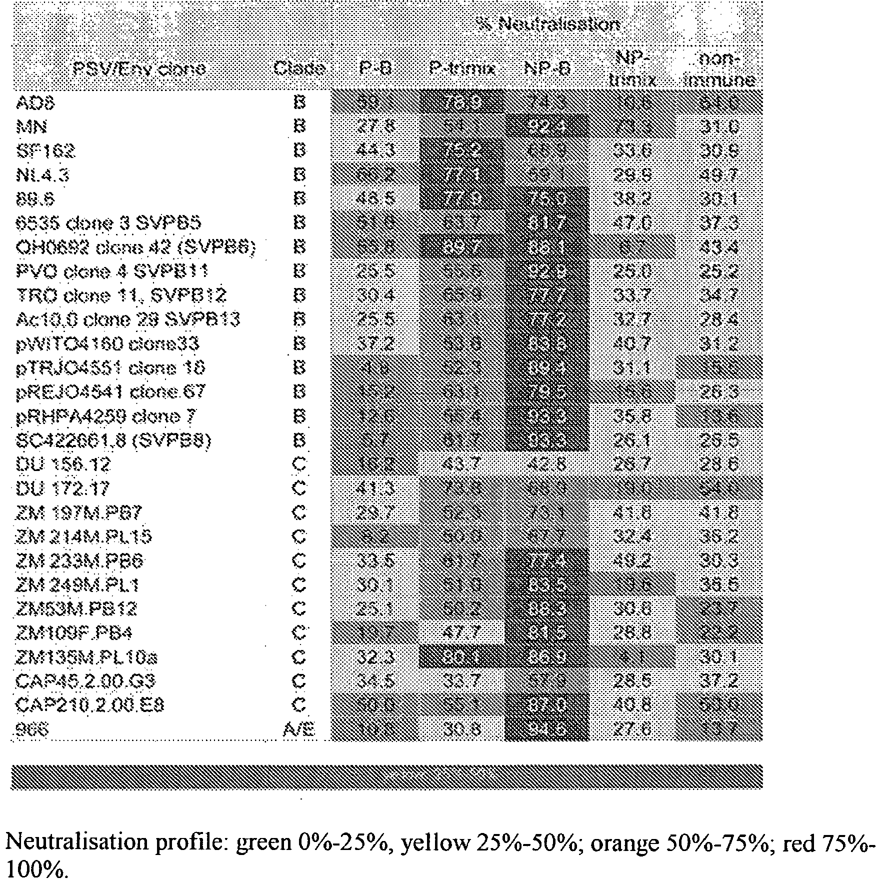

### EXAMPLE 12: Purified colostrum IgG has neutralizing activity.

Figure 7 shows the neutralizing activity of colostrum purified IgG from all 4 vaccinated cows for 2 EGFP Env-pseudotyped reporter viruses (clade B).The neutralization characteristic of En-pseudoyped viruses; AD^{*}; resistant, MN, sensitive.

## Claims

1. A non-therapeutic method for producing polyclonal antibodies or fragments thereof capable of binding to a human immunodeficiency virus (HIV) viral envelope (Env) protein or a fragment thereof, the method comprising:
immunizing a female cow with oligomeric Clade B gp140; and
obtaining the antibodies or fragments thereof from hyperimmune colostrum of the cow,
the method **characterised by** the steps of:
administering a primary vaccination of the oligomeric Clade B gp140 to the cow during a pregnancy; and
administering two booster vaccinations of the oligomeric Clade B gp140 to the cow during the pregnancy;
wherein the first booster vaccination is administered 7 weeks after the primary vaccination and the second booster vaccination is administered 12 weeks after the primary vaccination.

2. The non-therapeutic method of claim 1 wherein the oligomeric Clade B gp140 comprises gp140 trimers, dimers and monomers.

3. The non-therapeutic method of any one of claims 1-2, wherein the animal is immunized with the HIV viral envelope (Env) protein or fragment thereof and an adjuvant.

4. The non-therapeutic method of claim 3, wherein the adjuvant is a water-in-oil emulsion.

## Patentansprüche

1. Nicht-therapeutisches Verfahren zur Herstellung von polyklonalen Antikörpern oder Fragmenten davon, die zum Binden an ein HIV-(Humanimmunschwächevirus) virales Hüll- (Env) Protein oder ein Fragment davon imstande sind, wobei das Verfahren Folgendes umfasst:
Immunisieren einer weiblichen Kuh mit oligomerer Klade B gp140; und
Gewinnen der Antikörper oder Fragmente davon aus hyperimmunem Kolostrum der Kuh,
wobei das Verfahren durch die folgenden Schritte gekennzeichnet ist:
Verabreichen einer primären Impfung mit der oligomeren Klade B gp140 an die Kuh während einer Schwangerschaft; und
Verabreichen zweier Auffrischimpfungen mit der oligomeren Klade B gp140 an die Kuh während der Schwangerschaft;
worin die erste Auffrischimpfung 7 Wochen nach der primären Impfung verabreicht wird und die zweite Auffrischimpfung 12 Wochen nach der primären Impfung verabreicht wird.

2. Nicht-therapeutisches Verfahren nach Anspruch 1, worin die oligomere Klade B gp140 gp140-Trimere, -Dimere und -Monomere umfasst.

3. Nicht-therapeutisches Verfahren nach irgendeinem der Ansprüche 1-2, worin das Tier mit dem HIV-viralen Hüll- (Env) Protein oder Fragment davon und einem Adjuvant immunisiert wird.

4. Nicht-therapeutisches Verfahren Anspruch 3, worin das Adjuvant eine Wasser-in-Öl-Emulsion ist.

## Revendications

1. Procédé non thérapeutique permettant de produire des anticorps polyclonaux ou des fragments correspondants capables de se lier à une protéine de l'enveloppe virale (Env) du virus de l'immunodéficience humaine (VIH) ou à un fragment correspondant, le procédé consistant à :
immuniser une vache avec la protéine oligomérique gp140 de clade B ; et
obtenir les anticorps ou fragments correspondants à partir de colostrum hyperimmune de la vache,
le procédé étant **caractérisé par** les étapes consistant à :
administrer une vaccination primaire de la protéine oligomérique gp140 de clade B à la vache pendant une grossesse ; et
administrer deux vaccinations de rappel de la protéine oligomérique gp140 de clade B à la vache pendant la grossesse ;
la première vaccination de rappel étant administrée 7 semaines après la vaccination primaire, et la seconde vaccination de rappel étant administrée 12 semaines après la vaccination primaire.

2. Procédé non thérapeutique selon la revendication 1, dans lequel la protéine oligomérique gp140 de clade B comprend des trimères, des dimères et des monomères de gp140.

3. Procédé non thérapeutique selon l'une quelconque des revendications 1 à 2, dans lequel l'animal est immunisé avec la protéine de l'enveloppe virale (Env) du VIH ou un fragment correspondant et un adjuvant.

4. Procédé non thérapeutique selon la revendication 3, dans lequel l'adjuvant est une émulsion eau dans huile.
